(19) Europäisches Patentamt
European Patent Office
Office européen des brevets

(11) Veröffentlichungsnummer: **0 033 075**
**B1**

(12) **EUROPÄISCHE PATENTSCHRIFT**

(45) Veröffentlichungstag der Patentschrift: **23.03.83**

(51) Int. Cl.³: **C 07 C 49/747,** C 07 C 45/61, C 07 C 45/63, A 61 K 31/12

(21) Anmeldenummer: **81100135.3**

(22) Anmeldetag: **10.01.81**

(54) **Substituierte 1,8-Dihydroxy-9-(10H)anthracenone, Verfahren zu ihrer Herstellung und diese Verbindungen enthaltende Arzneimittel.**

(30) Priorität: **22.01.80 DE 3002089**

(43) Veröffentlichungstag der Anmeldung: **05.08.81 Patentblatt 81/31**

(45) Bekanntmachung des Hinweises auf die Patenterteilung: **23.03.83 Patentblatt 83/12**

(84) Benannte Vertragsstaaten: **AT BE CH DE FR GB IT LI LU NL SE**

(56) Entgegenhaltungen: **US-A-3 792 129**

(73) Patentinhaber: **Dr. Karl Thomae GmbH, Postfach 720, D-7950 Biberach (Riss) (DE)**

(72) Erfinder: **Brickl, Rolf, Dr., Dipl.-Chem., Erlenweg 37, D-7951 Warthausen 1 (DE)**
Erfinder: **Eberhardt, Hans, Dr., Dipl.-Chem., Ulrich-von Hutten-Weg 12, D-7950 Biberach 1 (DE)**

**EP 0 033 075 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Substituierte 1,8-Dihydroxy-9-(10H)anthracenone, Verfahren zu ihrer Herstellung und diese Verbindungen enthaltende Arzneimittel

Die vorliegende Erfindung betrifft substituierte 1,8-Dihydroxy-9-(10H)anthracenone der allgemeinen Formel I,

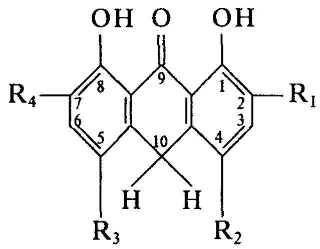

(I)

Verfahren zur ihrer Herstellung sowie diese Wirkstoffe enthaltende Arzneimittel.

In der obigen allgemeinen Formel I bedeuten $R_1$ bis $R_4$, die gleich oder verschieden sein können, Halogenatome, verzweigte Alkylgruppen oder Cycloalkylgruppen mit jeweils 3 bis 12 Kohlenstoffatomen oder einer bis drei der Reste $R_1$ bis $R_4$ auch ein Wasserstoffatom.

Unter den bei der Definition der Reste $R_1$ bis $R_4$ erwähnten Bedeutungen kommt somit für die Reste $R_1$ bis $R_4$ die Bedeutung des Wasserstoff-, Fluor-, Chlor-, Brom- oder Jodatoms, die der Isopropyl-, Isobutyl-, tert.Butyl-, Isopentyl-, Neopentyl-, tert.Pentyl-, 2-Methyl-pentyl-, 3-Methyl-pentyl-, 2,2-Dimethyl-butyl-, 2,3-Dimethyl-butyl-, 2-Methyl-hexyl-, 3-Methyl-hexyl-, 2,2-Dimethyl-pentyl-, 2,3-Dimethylpentyl-, 2,4-Dimethylpentyl-, 3,3-Dimethylpentyl-, 3-Äthyl-pentyl-, 2,2,3-Trimethylbutyl-, 2-Methyl-heptyl-, 2,5-Dimethyl-hexyl-, 2-Äthyl-hexyl-, 2-Methyl-octyl-, 2-Äthyl-heptyl-, 2-Methyl-nonyl-, 2-Äthyl-octyl-, 2-Methyl-decyl-, 2-Äthyl-nonyl-, 2-Propyl-heptyl-, 2-Methyl-undecyl-, 2-Äthyl-decyl-, 2-Propyl-octyl-, 2-Methyl-dodecyl-, 2-Äthyl-undecyl-, 2-Propyl-decyl-, 2-Butyl-nonyl-, 2-Pentylheptyl-, Cyclopropyl-, Cyclobutyl-, Cyclopentyl-, Cyclohexyl-, Cycloheptyl-, Cyclooctyl- oder Cyclododecylgruppe in Betracht.

Bevorzugte Verbindungen der obigen allgemeinen Formel I sind jedoch diejenigen, in der ein oder zwei der Reste $R_1$ bis $R_4$, die gleich oder verschieden sein können, Chlor- oder Bromatome, verzweigte Alkylgruppen oder Cycloalkylgruppen mit jeweils 3 bis 7 Kohlenstoffatomen und die übrigen der Reste $R_1$ bis $R_4$ Wasserstoffatome bedeuten.

Besonders bevorzugte Verbindungen der obigen allgemeinen Formel I sind jedoch diejenigen, in der ein oder zwei der Reste $R_1$ bis $R_4$, die gleich oder verschieden sein können, Chlor- oder Bromatome, Isopropyl-, tert.Butyl-, Isopentyl-, Isohexyl- oder Cyclohexylgruppen und die übrigen der Reste $R_1$ bis $R_4$ Wasserstoffatome darstellen.

Die Verbindungen der allgemeinen Formel I können wie folgt hergestellt werden:

a) Für die Herstellung von Verbindungen der allgemeinen Formel I, in der einer der Reste $R_1$ bis $R_4$ ein Halogenatom und der andere dieser Reste eine verzweigte Alkylgruppe oder eine Cycloalkylgruppe oder einer oder zwei der Reste $R_1$ bis $R_4$ eine verzweigte Alkylgruppe oder eine Cycloalkylgruppe mit jeweils 3 bis 12 Kohlenstoffatomen bedeuten und die übrigen Reste Wasserstoffatome sind:
Alkylierung eines 1,8-Dihydroxy-9-(10H)anthracenons der allgemeinen Formel II,

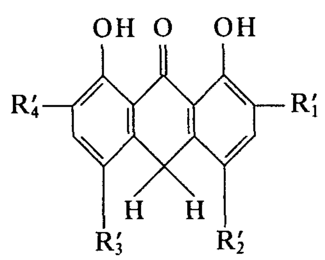

(II)

in der
$R_1'$ bis $R_4'$ Wasserstoffatome oder einer der Reste $R_1'$ bis $R_4'$ ein Halogenatom, eine verzweigte Alkylgruppe oder eine Cycloalkylgruppe mit jeweils 3 bis 12 Kohlenstoffatomen und die übrigen der Reste $R_1'$ bis $R_4'$ Wasserstoffatome darstellen, mit Alkenen, Cycloalkenen, Alkanolen oder Cycloalkanolen mit jeweils 3 bis 12 Kohlenstoffatomen in Gegenwart eines sauren Kondensationsmittels.
Die Alkylierung erfolgt entweder in Gegenwart von Säuren wie Phosphorsäure, Polyphosphorsäure, Schwefelsäure, Eisessig, Phosphoroxychlorid oder in Anwesenheit einer Lewis-Säure, wie

wasserfreies Aluminiumchlorid, Eisen(III)chlorid, Zinkchlorid, Phosphorpentoxid, Zinn(II)chlorid, Phosphorpentachlorid, in einem Lösungsmittel. Als Lösungsmittel dienen beispielsweise Äther, aromatische Kohlenwasserstoffe, wie Chlorbenzol, Nitrobenzol oder Phosphoroxychlorid oder halogenierte Kohlenwasserstoffe, wie Chloroform oder Äthylenchlorid. Die Umsetzung wird im allgemeinen bei Temperaturen zwischen 30 und 150° C durchgeführt.

b) Für die Herstellung von Verbindungen der allgemeinen Formel I, in der einer bis vier der Reste $R_1$ bis $R_4$ Halogenatome bedeuten und die übrigen Reste die eingangs erwähnten Bedeutungen besitzen:
Halogenierung eines 1,8-Dihydroxy-9-(10H)anthracenons der allgemeinen Formel II,

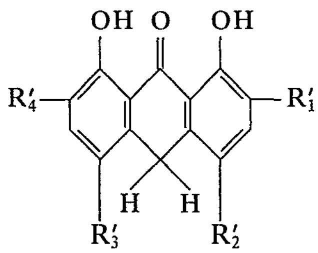

(II)

in der
$R_1'$ bis $R_4'$ Wasserstoffatome oder 1 bis 3 der Reste $R_1'$ bis $R_4'$ die für $R_1$ bis $R_4$ eingangs erwähnten Bedeutungen besitzen und die übrigen der Reste $R_1'$ bis $R_4'$ Wasserstoffatome darstellen.
Die Halogenierung erfolgt in einem geeigneten Lösungsmittel, beispielsweise in einem Äther, wie Diäthyläther oder Dioxan, in Eisessig, in Alkoholen, in einem halogenierten Kohlenwasserstoff, wie Chloroform oder Methylenchlorid oder in Mischungen dieser Lösungsmittel. Als Halogenierungsmittel dienen elementares Halogen, Sulfurylhalogenide oder Hypohalogenite, wie Natriumhypochlorit oder Trifluormethylhypochlorit.

Es wurde überraschenderweise gefunden, daß die Verbindungen der allgemeinen Formel I gegenüber der an sich bekannten Ausgangsverbindung der allgemeinen Formel II, in der $R_1'$ bis $R_4'$ Wasserstoffatome bedeuten, in bezug auf ihre pharmakologischen Eigenschaften besondere Vorteile aufweisen.

Das im vorliegenden Fall als Ausgangsprodukt dienende, synthetisch hergestellte 1,8-Dihydroxy-9-(10H)anthracenon (Dithranol, Anthralin) wurde 1916 von GALEWSKI in die externe Psoriasisbehandlung eingeführt. Vorher war seit 1878 das durch Extraktion aus dem Goa-Pulver der Pflanze Andira aroraba L. gewonnene Chrysarobin benutzt worden, das aus einem Gemisch verschiedener Substanzen besteht, als dessen wirksamer Bestandteil aber das 1,8-Dihydroxy-3-methyl-9-(10H)anthracenon angesehen wird. Da der Gehalt an Wirksubstanz im Goa-Pulver stets starken Schwankungen unterlag, war die Einführung des synthetisch hergestellten 1,8-Dihydroxy-9-(10H)anthracenons ein wesentlicher Therapiefortschritt. Seit dieser Zeit wird Dithranol weltweit mit gutem Erfolg zur Behandlung der Psoriasis eingesetzt. Wegen der sicheren Wirkung und fehlender systemischer Nebenwirkungen behauptet es auch noch heute seinen Platz als Standard-Therapeutikum zur Psoriasis-Behandlung.

Wesentliche Nachteile der Dithranol-Behandlung, die eine breite ambulante Anwendung stark einschränken, weshalb Dithranol meist an stationären Patienten in der Klinik angewendet wird, sind

a) die starke irreversible Verfärbung von Haut und Wäsche,
b) die stark hautreizende Wirkung von Dithranol, die nicht nur unangenehm ist, sondern bei unvorsichtiger Anwendungsweise durch Erzeugung eines Köbner-Phänomens zur Verschlimmerung der Psoriasis führen kann.

Untersuchungen zur Strukturspezifität von Dithranol, die vor allem von KREBS und SCHALTEGGER (Untersuchungen zur Strukturspezifität der Psoriasis-Heilmittel und Dithranol, Hautarzt, Seite 201, 1969) durchgeführt wurden, haben ergeben, daß für eine antipsoriatische Wirkung eine Minimal-Struktur folgender Art erforderlich ist:

a) Eine Hydroxygruppe in 1-Stellung;
b) ein Carbonal- respektive Anthron-Sauerstoff in 9-Stellung;
c) zwei freie H-Atome in 10-Stellung.

Wird in die Stellungen 2, 3 oder 4 eine OH-Gruppe eingeführt, so verschwindet die antipsoriatische Wirksamkeit. Von den ca. 30 von KREBS und SCHALTEGGER untersuchten Verbindungen zeigte keine Verbindung in der Psoriasis-Behandlung in bezug auf den therapeutischen Index eine überlegene Wirkung gegenüber Dithranol. Von HELLIER und WHITEFIELD wurde in England das 1,8,9-Triacetoxyanthracen (=Dithranoltriacetat) zur Psoriasis-Behandlung eingeführt. Dieses weist eine geringere Hautreizung auf, ist jedoch auch weniger wirksam als Dithranol, d. h., eine echte Verbesserung des therapeutischen Index wurde auch mit dieser Verbindung nicht erreicht.

Auch in der Arbeit von K. K. Mustakallio, Acta Dermatovener 59, Supplementum 85, Seite 125 ff. (1979), bei der zwei substituierte Dithranole verwendet wurden, konnte keine Verbesserung des therapeutischen Index erreicht werden: Bis-(formyläthyl)-dithranol zeigte zwar keine Reizung und Verfärbung, war aber auch antipsoriatisch unwirksam, 10-Acetyl-dithranol verfärbte und reizte die Haut in Konzentrationen ab 0,5% sogar stärker als die Ausgangsverbindung.

Aufgabe der vorliegenden Erfindung war es deshalb, die Nachteile der Dithranol-Therapie unter Erhalt der antipsoriatischen Wirksamkeit zu beseitigen. Die weiter unten genauer beschriebenen Verbindungen weisen gegenüber der Ausgangsverbindung Dithranol und dem Naturprodukt Chrysarobin folgende wesentliche Vorteile auf:

1. Keine Verfärbung der Haut oder der Wäsche;
2. erheblich geringere Hautreizung;
3. erheblich geringere Toxizität;
4. erheblich bessere chemische Stabilität auch bei Belichtung, die die Möglichkeit bietet, galenisch stabile Zubereitungen herzustellen;
5. geringere Eigenfarbe, d. h., die Substanz und damit auch deren Zubereitungen sind nicht so stark gelb gefärbt.

Die Verbindungen hemmen wie Dithranol Schlüsselenzyme des Kohlenhydratstoffwechsels und verlangsamen somit beschleunigte Zellteilungsvorgänge. Diese Verbindungen sind deshalb zur Behandlung von Hautkrankheiten wie Psoriasis, Kopfschuppen, Ichthyosis und hyperkeratotischen Hautzuständen geeignet.

So wurden die bekannten Verbindungen

| | |
|---|---|
| 1,8-Dihydroxy-9-(10H)anthracenon (Dithranol) | = A |
| und | |
| 1,8-Dihydroxy-3-methyl-9-(10H)anthracenon (Chrysarobin) | = B |

gegenüber den neuen Verbindungen

| | |
|---|---|
| 4,5-Dichlor-1,8-dihydroxy-9-(10H)anthracenon | = C |
| 2-tert.Butyl-1,8-dihydroxy-9-(10H)anthracenon | = D |
| 2-(2-Hexyl)-1,8-dihydroxy-9-(10H)anthracenon | = E |
| 1,8-Dihydroxy-4-isopropyl-9-(10H)anthracenon | = F |
| 1,8-Dihydroxy-2,4-diisopropyl-9-(10H)anthracenon | = G |
| 4-Chlor-1,8-dihydroxy-9-(10H)anthracenon | = H |
| 2-Cyclohexyl-1,8-dihydroxy-9-(10H)anthracenon | = I |
| 2,7-Di-tert.butyl-1,8-dihydroxy-9-(10H)anthracenon | = K |

in bezug auf ihre Hemmwirkung gegenüber Enzymen, ihre irritierenden Eigenschaften, ihre Verfärbung von Haut und Wäsche und ihre Toxizität vergleichend getestet.

Die Hemmwirkung gegenüber Enzymen wurde durch die Messung der Hemmung der Glucose-6-phosphatdehydrase ermittelt.

Beobachtet wird das Gleichgewicht:

$$\text{Glucose-6-phosphat} + \text{NADP}^+ \xrightarrow{\text{G 6 P-DH}} \text{Gluconsäure-6-phosphat} + \text{NADPH} + \text{H}^+$$

(NADP = Nikotinamid-adenin-dinukleotid-phosphat).

Die Bildungsgeschwindigkeit von NADPH ist ein Maß für die Enzymaktivität; sie kann anhand der Extinktionszunahme bei 340, 334 oder 366 mm pro Zeiteinheit verfolgt werden.

## Methodik

0,025 ml Glucose-6-phosphat-dehydrogenase (Fa. Boehringer, Mannheim) werden mit destilliertem Wasser auf 10 ml aufgefüllt (Lösung I). 100 mg Nikotinamid-adenin-dinukleotid-phosphat werden in 13 ml dest. Wasser gelöst (Lösung II). 47,2 mg Glucose-6-phosphat löst man in weiteren 10 ml dest. Wasser (Lösung III). Nebenher wird eine Pufferlösung (Lösung IV) wie folgt bereitet:

0,28 g Triäthanolamin-hydrochlorid und 1,461 g Äthylendiamintetraessigsäure-dinatriumsalz werden

in 1 l dest. Wasser gelöst und mit Natronlauge auf pH 7,6 eingestellt. Die zu untersuchende Substanz wird in Dimethylformamid oder Äthanol gelöst (Lösung V). Geprüfte Endkonzentrationen: 25; 12,5; 6,25; 3,125; 1,56 und 0,78 µg/ml.

Bestimmung der Inkubationshemmung

0,1 ml Lösung I, 0,1 ml Lösung II, 2,67 ml Lösung IV und 0,03 ml Lösung V werden 120 Minuten bei 37°C gehalten. Dann wird 0,1 ml Lösung III zugegeben, durchmischt und die Extinktionsänderung bei 366 nm über 3 Minuten spektralphotometrisch gemessen. Die Hemmwerte werden aus den Mittelwerten dreier Messungen (als Extinktionsänderung pro Minute) im Vergleich zu Kontrollen, bei denen als Inhibitorlösung das reine Lösungsmittel zugesetzt wird, berechnet. Dann wird aus den Hemmwerten für die unterschiedlichen Konzentrationen die $ED_{50}$ nach der Methode von Reed und Muench berechnet.

Die nachfolgende Tabelle enthält die so bestimmten Werte:

Tabelle 1

| Verbindung | $ED_{50}$ [µg/ml]<br>Inkubationshemmung nach 120 Minuten |
|---|---|
| A | 5,60 |
| B | 6,86 |
| C | 4,63 |
| D | 6,32 |
| E | 3,61 |
| F | 5,35 |
| G | 5,67 |
| H | 3,85 |
| I | 3,94 |
| K | 7,77 |

Man sieht, daß die neuen Verbindungen etwa gleiche, in manchen Fällen etwas bessere Wirkung als die bekannten Verbindungen A und B aufweisen.

Bestimmung der akuten Toxizität

Die akute Toxizität wurde an Maus und Ratte ermittelt. Bestimmt wurde die $LD_{50}$, d. h. die Dosis, nach deren Verabreichung innerhalb von 14 Tagen 50% der Tiere verstarben bzw. es wird angegeben, wieviel von den 10 Tieren innerhalb dieses Beobachtungszeitraumes verstorben sind. Die Ergebnisse sind in der Tabelle 2 zusammengefaßt.

Tabelle 2

| Tierart | Applikationsart | Verbindung A | | | Verbindung F | | | Verbindung C | | Verbindung K | |
|---|---|---|---|---|---|---|---|---|---|---|---|
| | | $LD_{50}$ mg/kg | Dosis mg/kg | Anzahl der verstorbenen Tiere | $LD_{50}$ mg/kg | Dosis mg/kg | Anzahl der verstorbenen Tiere | Dosis mg/kg | Anzahl der verstorbenen Tiere | Dosis mg/kg | Anzahl der verstorbenen Tiere |
| Maus | i.p. | 2,42 | – | – | 52 | – | – | 2000 | 1/10 | 1000 | 0/10 |
| Maus | sc. | 2,22 | – | – | – | 2000 | 0/10 | 2000 | 6/10 | 1000 | 0/10 |
| Maus | p.o. | – | 2000 | 2/10 | – | 2000 | 0/10 | 2000 | 0/10 | 4000 | 0/10 |
| Ratte | i.p. | 5,19 | – | – | 45 | – | – | 2000 | 0/10 | | |
| Ratte | sc. | – | 1000 | 2/10 | – | 2000 | 0/10 | 2000 | 0/10 | | |
| Ratte | p.o. | – | 2000 | 1/10 | – | 2000 | 0/10 | 2000 | 0/10 | | |

Die Untersuchungen zur Hautverträglichkeit wurden am Meerschweinchen und orientierend auch am Menschen durchgeführt.

Methodik der Verträglichkeit am Meerschweinchen

Meerschweinchen vom Stamm Perbright White, ca. 350 g schwer, werden mit Fertigfutter Altromin 3022 und Wasser ad lib. gefüttert.

Während des Versuches sind die Tiere in Einzelkäfigen untergebracht.

Die zu prüfenden Substanzen werden, in einem gut verträglichen Lösungsmittel gelöst, 3 Wochen lang, montags bis freitags täglich, in einem Testfeld mit Durchmesser 3,5 cm auf die Haut des Tieres aufgetragen. Die Tiere werden jeweils montags, mittwochs und freitags am frühen Vormittag rasiert, die Substanzbehandlung erfolgt am frühen Nachmittag. Die Beurteilung der Hautreaktionen erfolgt direkt vor der Substanz-Applikation. Die Beurteilung erfolgt visuell und berücksichtigt Erytheme, Ödeme und Mykosen der Haut. Es wird wie folgt bewertet:

| | |
|---|---|
| 0 | keine Reizung |
| 0,5 | schwaches Erythem |
| 1 | deutliches Erythem |
| 2 | starkes Erythem mit beginnendem Ödem |
| 3 | sehr starkes Erythem mit Ödem oder beginnenden Nekrosen |

Zur Gesamtbeurteilung einer Tiergruppe wird die Summe der Reaktionen herangezogen.

Zur Prüfung auf sensibilisierende Wirkung wird 2 Wochen nach Ende der Hautverträglichkeit mit 1/10 bis 1/50 der ursprünglich angewendeten Konzentration hinter dem Ohr auf Sensibilisierung getestet. Die Ergebnisse der Hautverträglichkeit zeigt Tabelle 3.

Tabelle 3

| Verbindung | Konzentration | Anzahl der Tiere | Summe der Reaktionen geteilt durch Anzahl der Tiere am Ende der | | |
|---|---|---|---|---|---|
| | % | | 1. | 2. | 3. Woche |
| A | 0,015 | 6 | 0,66 | 0,33 | 0,33 |
| | 0,03 | 12 | 1,3 | 2,16 | 2,25 |
| C | 0,03 | 12 | 0 | 0 | 0,04 |
| | 0,045 | 6 | 0,166 | 0,66 | 0,41 |
| D | 0,03 | 12 | 0,083 | 0,042 | 0,00 |
| F | 0,015 | 6 | 0,085 | 0,085 | 0,085 |
| | 0,045 | 6 | 0,085 | 0,085 | 0,085 |
| K | 0,03 | 12 | 0,00 | 0,042 | 0,00 |

Man sieht, daß die neuen Verbindungen C, F, D und K eine erheblich bessere Hautverträglichkeit zeigen als die Verbindung A.

Bei Verbindung A wurden bei Anwendung von 0,03% Substanz 3 von 12 Tieren sensibilisiert. Die Verbindungen C, D, F und K zeigten keine Sensibilisierung.

## Orientierende Hautverträglichkeit am Menschen

### Methodik

Salben, die jeweils 0,44% der Verbindungen A, C, D, E, F, G und K enthielten, wurden auf den Unterarm einer freiwilligen Versuchsperson aufgetragen, mit Verbandgaze abgedeckt und 16 Stunden auf der Haut belassen. Nach 16 Stunden wurden die Zubereitungen mit Seifenlösung abgewaschen, die Hautreizung und Verfärbung visuell beurteilt und fotografiert. Anschließend wurde erneut Zubereitung aufgetragen, die bis zum nächsten Tag auf der Haut belassen wurde. Anschließend wurden wieder die Reaktionen beurteilt und erneut behandelt. Nach 3 Tagen (3maliger Anwendung) wurde die Konzentration bei den Zubereitungen, die keine Reaktionen hervorriefen, auf 0,88% verdoppelt, und es erfolgte eine weitere Behandlung über 2 Tage.

### Ergebnisse

Während die Zubereitung mit Dithranol (Verbindung A) schon nach der zweiten Anwendung eine starke Hautreizung mit Erythem und eine braunviolette Verfärbung zeigte, wurde bei den Verbindungen C, D, E, F und G keinerlei Hautreizung oder Verfärbung der Haut festgestellt. Die Verbandgaze, mit der das Behandlungsfeld abgedeckt worden war, war bei Verbindung A irreversibel violett verfärbt, während bei den Verbindungen C, D, E, F, G und K keine Verfärbung festzustellen war. Das mit Verbindung A behandelte Hautareal schälte sich nach ca. 1 Woche, während bei der mit den anderen Verbindungen behandelten Haut keine weiteren Veränderungen festzustellen waren.

Die Verbindung B wirkt, wie ein Auftragen einer 0,44%igen Salbe zeigte, ähnlich hautverfärbend wie die Verbindung A.

In einem weiteren Versuch wurden analog der Methode von Mustakallio die Verbindung A in den Konzentrationen 1, 2, 4, 8, 16, 32 mMol, die Verbindungen C und K hingegen in den Konzentrationen 4, 8, 16, 32, 64, 128 mMol in weißer Vaseline aufgetragen und 4 bzw. 16 Stunden unter Okklusion auf der Haut belassen. Während bei Verbindung A nach 4 Stunden Okklusion mit Konzentrationen ab 2 mMol, nach 16 Stunden ab 1 mMol Verfärbungen und Hautreizungen auftraten, konnten bei den Verbindungen C und K auch in den höchsten Konzentrationen weder nach 4 noch nach 16 Stunden irgendwelche Hautreizungen oder Verfärbungen beobachtet werden. Dies bedeutet, daß die neuen Verbindungen zumindest um den Faktor 64 besser verträglich sind.

### Penetration und Metabolisierung in der Haut

Bei Penetrationsversuchen in vitro in Schweinehaut (diese wurde verwendet, da sie im Hinblick auf den morphologischen Aufbau und Behaarung der menschlichen Haut relativ ähnlich ist) konnte mit Hilfe der Dünnschichtchromatographie nachgewiesen werden, daß die Verbindungen A, C, G und K aus der gleichen Grundlage etwa gleich stark in die verschiedenen Hautschichten penetrieren und in ähnlicher Weise metabolisiert werden.

Dies beweist, daß die wesentlich bessere Hautverträglichkeit der erfindungsgemäßen Verbindungen nicht auf schlechtere Penetration zurückzuführen ist.

### Eigenfarbe der Substanz

Bei topisch aufzutragenden Substanzen stört Farbigkeit aus kosmetischen Gründen und auch wegen der möglichen Wäscheverschmutzung. Abb. 1 zeigt, daß die Verbindungen C und K im kurzwelligen Teil des sichtbaren Lichtes (ab 380 nm) erheblich weniger UV-Absorption zeigen, d. h., sie sind schwächer gelb gefärbt. Dies kommt auch vom optischen Eindruck her in Zubereitungen zum Ausdruck. Da außerdem keine Verfärbung auftritt, sind leichte Wäscheverschmutzungen im Gegensatz zu Dithranol wie durch Wäscheversuche gezeigt werden konnte, wieder leicht entfernbar.

### Photostabilität und Photosensibilität

Da Antipsoriatika auch auf lichtexponierte Hautstellen aufgetragen werden, wurde die Stabilität bei Bestrahlung besonders bei dihalogenierten Derivaten, bei denen bei Belichtung die Radikalbildung denkbar ist, untersucht.

### Stabilität bei Bestrahlung

C und Dithranol (A) wurden in Lösung (Toluol bzw. Xylol) mit einer 400-W-Metallhalogendampflampe, Typ HPM 12, Fa. Phillips, 30 Minuten bestrahlt. Während Dithranol unter diesen Bedingungen nahezu vollständig zersetzt wird, konnte bei C keine Zersetzung im Dünnschichtchromatogramm nachgewiesen werden. Außerdem konnte gaschromatographisch keine Chlorierung von Toluol, die auf eine Radikalbildung der Substanz deuten würde, nachgewiesen werden (Empfindlichkeit 0,0001%).

### Photosensibilisierung

Da manche halogenierte Aromaten photosensibilisierend wirken, wurde auch die Photosensibilisierung von Verbindung C in vivo am Meerschweinchen getestet.

### Methodik

Meerschweinchen wurden 3 Wochen lang an 5 Wochentagen mit 0,01%iger alkoholischer Lösung von C behandelt. Nach 30 Minuten wurde jeweils 10 Minuten lang mit einer 125-W-Quecksilber-Hochdrucklampe (Höhensonne) bestrahlt. (Die Bestrahlung wurde so gewählt, daß noch kein UV-B-Erythem auftrat.)

Es konnte keine photosensibilisierende Wirkung nachgewiesen werden.

Die folgenden Beispiele sollen die Erfindung noch näher erläutern:

### Beispiel 1

4,5-Dichlor-1,8-dihydroxy-9-(10H)anthracenon

In eine Mischung von 2,23 l Eisessig, 1,11 l Chloroform und 0,44 l Äthanol werden bei 0 bis −5° C 235 g Chlor (3,3 Mol) eingeleitet. Anschließend werden portionsweise ebenfalls bei 0 bis −5° C 250 g 1,8-Dihydroxy-9-(10H)anthracenon portionsweise zugegeben. Anschließend wird noch 20 Minuten nachgerührt. Die entstandene Dichlorverbindung wird abgesaugt, mit Petroläther und Chloroform nachgewaschen und aus Toluol umkristallisiert.

F.: 264° C, Ausbeute 156 g (48% der Theorie).

Auf diese Weise wurde hergestellt:

4,5-Dibrom-1,8-dihydroxy-9-(10H)anthracenon,
Schmelzpunkt: 239° C, Ausbeute: 62% der Theorie.

In analoger Weise, aber mit 1,5 Mol Halogen, die in eine Suspension von 1,8-Dihydroxy-9-(10H)anthracenon im genannten Lösungsmittelgemisch eingeleitet werden, wurden hergestellt:

4-Chlor-1,8-dihydroxy-9-(10H)anthracenon,
Schmelzpunkt: 178° C, Ausbeute: 32% (Ausbeute an 4,5-Dichlorverbindung 28%).
4-Brom-1,8-dihydroxy-9-(10H)anthracenon,
Schmelzpunkt: 169° C, Ausbeute: 59% der Theorie (Ausbeute an 4,5-Dibromverbindung 20%).

Setzt man 4-tert.Butyl-1,8-dihydroxy-9-(10H)anthracenon in analoger Weise mit Brom um, so entstehen

bei Verwendung von 1,5 Mol Brom als Hauptprodukt
4-Brom-5-tert.butyl-1,8-dihydroxy-9-(10H)anthracenon,
F. = 236 − 238° C, Ausbeute: 55%;
bei Verwendung von 3 Mol Brom als Hauptprodukt
2,4-Dibrom-5-tert.butyl-1,8-dihydroxy-9-(10H)anthracenon,
F. = 160 − 162° C, Ausbeute: 62%;
bei Verwendung eines großen Bromüberschusses (ca. 8 Mol) eine Tribromverbindung von 4-tert.Butyl-1,8-dihydroxy-9-(10H)anthracenon vom F. 232 − 234° C.

Die Stellung der Bromatome konnte wegen zu geringer Löslichkeit nicht bestimmt werden, vermutlich sind die Positionen 2, 4 und 7 besetzt.

Beispiel 2

Mischung aus 2-Isopropyl-1,8-dihydroxy-9-(10H)anthracenon und 4-Isopropyl-1,8-dihydroxy-9-(10H)anthracenon

Eine Lösung von 56 g 1,8-Dihydroxy-9-(10H)anthracenon in 45 ml Isopropanol und 400 ml Phosphorsäure (105%ig) wird für 2 Stunden bei 110° C gerührt, dann abgekühlt und auf Eiswasser gegossen. Die ausfallende Substanz wird abgesaugt und über eine Kieselgelsäule (Laufmittel Chloroform/Heptan 1 : 1) aufgetrennt. Es werden 49,5 g einer Substanz vom Schmelzpunkt 109 – 110° C erhalten, die sich dünnschichtchromatographisch als einheitlich zeigt und deren Elementaranalyse den berechneten Werten für eine Monoisopropylverbindung von 1,8-Dihydroxy-9-anthracenon entspricht. Die Strukturaufklärung mit Hilfe der 13C-NMR-Spektroskopie ergab, daß es sich um eine Mischung, die aus 15% 2-Isopropyl- und aus 85% der 4-Isopropylverbindung besteht, handelt. Die Ausbeuten, bezogen auf die Ausgangsverbindung, beträgt 72%. Die 4-Isopropylverbindung läßt sich durch Umkristallisation aus n-Heptan isolieren.
F. = 115 – 117° C.

In analoger Weise wurden aus 1,8-Dihydroxy-9-(10H)anthracenon und den entsprechenden sekundären Alkoholen folgende Verbindungen hergestellt:

4-tert.Butyl-1,8-dihydroxy-9-(10H)anthracenon,
Schmelzpunkt: 126 – 127° C;
4-Isohexyl-1,8-dihydroxy-9-(10H)anthracenon,
Schmelzpunkt: 65° C;
2-Cyclohexyl-1,8-dihydroxy-9-(10H)anthracenon,
Schmelzpunkt: 145 – 147° C.

Als Nebenprodukte bei diesen Umsetzungen entstehen auch Dialkylverbindungen, z. B. 2,4-Diisopropyl-1,8-dihydroxy-9-(10H)anthracenon und 2,7-Diisopropyl-1,8-dihydroxy-9-(10H)anthracenon und 2,7-Di-tert.butyl-1,8-dihydroxy-9-(10H)anthracenon.

In analoger Weise wurden noch hergestellt

aus 4-Brom-1,8-dihydroxy-9-(10H)anthracenon das
4-Brom-5-isopropyl-1,8-dihydroxy-9-(10H)anthracenon vom F. = 126 – 130° C.

Das Reaktionsprodukt enthielt noch 25% an 4-Brom-7-isopropyl-1,9-dihydroxy-9-(10H)anthracenon. Aus 4-Chlor-1,8-dihydroxy-9-(10H)anthracenon wurde das

4-Chlor-5-isopropyl-1,8-dihydroxy-9-(10H)anthracenon vom F. = 120 – 123° C

hergestellt.

Beispiel 3

2-tert.Butyl-1,8-dihydroxy-9-(10H)anthracenon

In eine Lösung von 11,3 g 1,8-Dihydroxy-9-(10H)anthracenon in 100 ml Chloroform und 6 ml konzentrierter Schwefelsäure werden unter Kochen am Rückfluß ca. 7 g Isobutylen eingeleitet. Dann wird auf Wasser gegossen und die organische Phase abgetrennt. Die weitere Aufarbeitung erfolgt analog Beispiel 2. Es werden 3,9 g 2-tert.Butyl-1,8-dihydroxy-9-(10H)anthracenon erhalten. Dies entspricht 27% der Theorie.
F.: 126 – 127° C.

In analoger Weise wurden auch die folgenden Alkylverbindungen durch Umsetzen mit den entsprechenden Alkenen erhalten:

2-Isohexyl-1,8-dihydroxy-9-(10H)anthracenon,
F.: 65° C;
2-Cyclohexyl-1,8-dihydroxy-9-(10H)anthracenon,
F.: 145 – 147° C;
2-Isopropyl-1,8-dihydroxy-9-(10H)anthracenon,
F.: 115 – 117° C;
Gemisch aus 2-Dodecyl-1,8-dihydroxy-9-(10H)anthracenon und
4-Dodecyl-1,8-dihydroxy-9-(10H)anthracenon,
$R_f$-Wert: 0,59 (Merck DC-Fertigplatten 60 F-254, Laufmittel: Toluol ohne Kammersättigung);
zum Vergleich: 1,8-Dihydroxy-9-(10H)anthracenon, $R_f$ = 0,53 (gleiche Bedingungen).

Beispiel 4

2,7-Di-tert.butyl-1,8-dihydroxy-9-(10H)anthracenon

In eine Lösung von 22,6 g 1,8-Dihydroxy-9-(10H)anthracenon in 150 ml Eisessig, 75 ml Chloroform und 90 ml konzentrierter Schwefelsäure werden unter Rühren bei 60°C innerhalb 3 Stunden ca. 50 g Isobutylen eingeleitet. Dann kühlt man auf 5°C ab und saugt die auskristallisierte Substanz, die bereits einen hohen Reinheitsgrad aufweist, ab. Das zu ca. 5–8% enthaltene 2-tert.Butyl-1,8-dihydroxy-9-(10H)anthracenon wird durch Rühren mit einer 1 : 1-Chloroform/Eisessigmischung entfernt und kann wieder eingesetzt werden.
Ausbeute: 35% der Theorie, Schmelzpunkt: 192°C.

In analoger Weise lassen sich auch die anderen disubstituierten Alkylverbindungen aus den entsprechenden Alkenen herstellen.

Die Verbindungen der allgemeinen Formel I lassen sich in die üblichen pharmazeutischen Zubereitungsformen, beispielsweise in Salben, Gels, Cremes etc., einarbeiten. Die Zubereitungsformen enthalten im allgemeinen die Wirkstoffe in einer Konzentration zwischen 0,01 und 5%, vorzugsweise 0,1 bis 1%. Die Herstellung solcher Zubereitungsformen erfolgt beispielsweise wie folgt:

Beispiel I

Salbe, enthaltend 4,5-Dichlor-1,8-dihydroxy-9(10H)anthracenon
in einer Konzentration von 0,5 Gew.-%

Zusammensetzung:

100 g Salbe enthalten:

| | |
|---|---|
| Wirksubstanz | 0,50 g |
| tert.-Butyl-hydroxy-anisol | 0,30 g |
| Salicylsäure | 1,00 g |
| Cetylalkohol | 10,00 g |
| Paraffinöl, dickflüssig | 45,00 g |
| Vaseline weiß | 43,20 g |
| | 100,00 g |

Herstellung:

Die Bestandteile werden zusammengegeben, das Gemisch anschließend unter Rühren auf 70°C erwärmt und so lange weitergerührt, bis die Temperatur auf 40°C abgekühlt ist. Hernach kann die Salbe abgefüllt werden.

Beispiel II

Gel, enthaltend 0,5 Gew.-% 4,5-Dichlor-1,8-dihydroxy-9(10H)anthracenon

Zusammensetzung:

100 g Gel enthalten

| | |
|---|---|
| Wirksubstanz | 0,50 g |
| Vaseline gelb | 78,50 g |
| Paraffinöl, dickflüssig | 21,00 g |
| | 100,00 g |

Herstellung:

erfolgt analog dem Beispiel I.

## Beispiel III

Lipo-Gel, enthaltend 0,5 Gew.-% 4,5-Dichlor-1,8-dihydroxy-9(10H)anthracenon

Zusammensetzung:

100 g Lipo-Gel enthalten

| | |
|---|---|
| Wirksubstanz | 0,50 g |
| Propan-1,2-diol-fettsäureester mittlerer Kettenlänge (Miglyol 840) | 82,50 g |
| Hydriertes Rizinusöl | 15,00 g |
| Triäthylcitrat | 2,00 g |
| | 100,00 g |

Herstellung:

erfolgt analog dem Beispiel I.

## Beispiel IV

Creme, enthaltend 0,2 Gew.-% 4,5-Dichlor-1,8-dihydroxy-9-(10H)anthracenon

100 g Creme enthalten:

| | |
|---|---|
| Wirkstoff | 0,20 g |
| Cetylstearylalkohol | 12,80 g |
| Propan-1,2-diolfettsäureester mittlerer Kettenlänge (Miglyol 840) | 25,00 g |
| Cremophor O (acylsubstituiertes Polyadditionsprodukt von Cetylstearylalkohol mit 20 bis 25 Mol Äthylenoxid) | 3,00 g |
| Span 40 (Sorbitanmonopalmitat) | 3,00 g |
| Wasser dest. | 56,00 g |
| | 100,00 g |

Herstellung:

Span 40, Cremophor O, Miglyol 840, Cetylstearylalkohol werden bei 75° C zusammengeschmolzen und bei dieser Temperatur gehalten, der Wirkstoff wird eingerührt und aufgekochtes Wasser unter Rühren bei 75° C zugegeben. Das fertige Gemisch füllt man anschließend ab und läßt abkühlen.

## Beispiel V

Fettcreme, enthaltend 0,2 Gew.-% 4,5-Dichlor-1,8-dihydro-9-(10H)anthracenon

100 g Creme enthalten:

| | |
|---|---|
| Wirkstoff | 0,20 g |
| Propan-1,2-diol-fettsäureester mittlerer Kettenlänge (Miglyol 840) | 20,80 g |
| Nichtionogener Emulgator | 7,00 g |
| Sorbitensesquioleat | 7,00 g |
| Miglyol Gel | 15,00 g |
| Wasser | 50,00 g |
| | 100,00 g |

Herstellung:

erfolgt analog dem Beispiel IV.

**Patentansprüche**

1. Substituierte 1,8-Dihydroxy-9-(10H)anthracenone der allgemeinen Formel I,

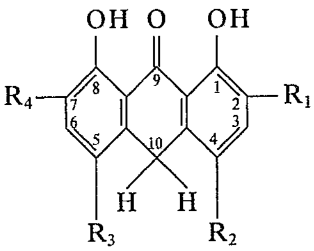

(I)

in der

$R_1$ bis $R_4$, die gleich oder voneinander verschieden sein können, Halogenatome, verzweigte Alkylgruppen oder Cycloalkylgruppen mit jeweils 3 bis 12 Kohlenstoffatomen oder einer bis drei der Reste $R_1$ bis $R_4$ auch ein Wasserstoffatom bedeuten.

2. 1,8-Dihydroxy-9-(10H)anthracenone der allgemeinen Formel I gemäß Anspruch 1, in der ein oder zwei der Reste $R_1$ bis $R_4$, die gleich oder verschieden sein können, Chlor- oder Bromatome, verzweigte Alkylgruppen mit 3 bis 7 Kohlenstoffatomen oder Cycloalkylgruppen mit 5 bis 7 Kohlenstoffatomen und die übrigen der Reste $R_1$ bis $R_4$ Wasserstoffatome bedeuten.

3. 1,8-Dihydroxy-9-(10H)anthracenone der allgemeinen Formel I gemäß Anspruch 2, in der ein oder zwei der Reste $R_1$ bis $R_4$, die gleich oder verschieden sein können, Chlor- oder Bromatome, Isopropyl-, Isopentyl-, Hexyl-(2)- oder Cyclohexylgruppen und die übrigen der Reste $R_1$ bis $R_4$ Wasserstoffatome darstellen.

4. 4,5-Dichlor-1,8-dihydroxy-9-(10H)anthracenon.

5. 2-tert.Butyl-1,8-dihydroxy-9-(10H)anthracenon.

6. 2,7-Di-tert.butyl-1,8-dihydroxy-9-(10H)anthracenon.

7. 1,8-Dihydroxy-4-isopropyl-9-(10H)anthracenon.

8. Arzneimittel, enthaltend eine oder mehrere Verbindungen der allgemeinen Formel I gemäß Anspruch 1 neben üblichen Träger- und Hilfsstoffen.

9. Verwendung von Verbindungen der allgemeinen Formel I gemäß Anspruch 1 zur Herstellung von Arzneimitteln auf nichtchemischem Weg.

10. Arzneimittel gemäß Anspruch 8 zur Behandlung von Hautkrankheiten wie Psoriasis, Kopfschuppen, Ichthyosis und hyperkeratotischen Hautzuständen.

11. Verfahren zur Herstellung neuer substituierter 1,8-Dihydroxy-9-(10H)anthracenone der allgemeinen Formel I,

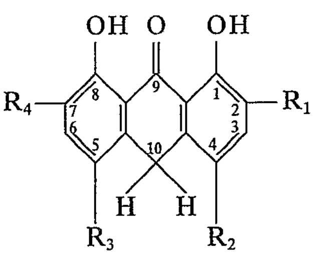

(I)

in der

$R_1$ bis $R_4$, die gleich oder verschieden sein können, Halogenatome, verzweigte Alkylgruppen oder Cycloalkylgruppen mit jeweils 3 bis 12 Kohlenstoffatomen oder einer bis drei der Reste $R_1$ bis $R_4$ auch ein Wasserstoffatom bedeuten, dadurch gekennzeichnet, daß

a) für die Herstellung von Verbindungen der allgemeinen Formel I, in der einer der Reste $R_1$ bis $R_4$ ein Halogenatom und der andere dieser Reste eine verzweigte Alkylgruppe oder eine gesättigte Cycloalkylgruppe oder einer oder zwei der Reste $R_1$ bis $R_4$ eine verzweigte Alkylgruppe oder eine gesättigte Cycloalkylgruppe mit jeweils 3 bis 12 Kohlenstoffatomen bedeuten und die übrigen Reste Wasserstoffatome sind, ein 1,8-Dihydroxy-9-(10H)anthracenon der allgemeinen Formel II,

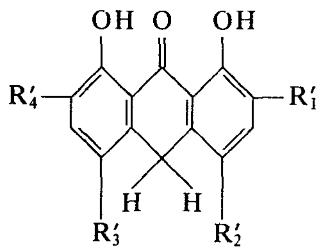

(II)

in der
$R_1'$ bis $R_4'$ Wasserstoffatome oder einer der Reste $R_1'$ bis $R_4'$ ein Halogenatom, eine verzweigte Alkylgruppe oder eine Cycloalkylgruppe mit jeweils 3 bis 12 Kohlenstoffatomen und die übrigen der Reste $R_1'$ bis $R_4'$ Wasserstoffatome darstellen, mit Alkenen, Cycloalkenen, Alkanolen oder Cycloalkanolen mit jeweils 3 bis 12 Kohlenstoffatomen, bei Temperaturen zwischen 30 und 150°C in Gegenwart saurer Agenzien in einem Lösungsmittel umgesetzt wird, oder

b) für die Herstellung von Verbindungen der allgemeinen Formel I, in der einer bis vier der Reste $R_1$ bis $R_4$ Halogenatome bedeuten und die übrigen Reste die eingangs erwähnten Bedeutungen besitzen, ein 1,8-Dihydroxy-9-(10H)anthracenon der allgemeinen Formel II,

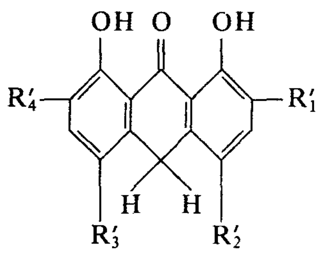

(II)

in der
$R_1'$ bis $R_4'$ Wasserstoffatome oder 1 bis 3 der Reste $R_1'$ bis $R_4'$ die für $R_1$ bis $R_4$ eingangs erwähnten Bedeutungen besitzen und die übrigen der Reste $R_1'$ bis $R_4'$ Wasserstoffatome darstellen, in einem Lösungsmittel halogeniert wird.

12. Verfahren gemäß Anspruch 11b, dadurch gekennzeichnet, daß als Halogenierungsmittel freies Halogen, Sulfurylhalogenide oder Hypohalogenite Verwendung finden.

## Claims

1. Substituted 1,8-dihydroxy-9-(10H)-anthracenones of general formula I,

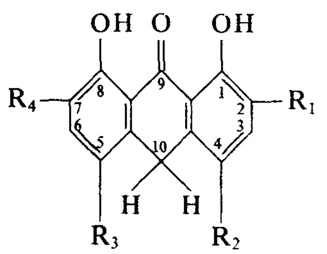

(I)

wherein
$R_1$ to $R_4$, which may be the same as or different from one another, represent halogen atoms, branched alkyl groups or cycloalkyl groups each with 3 to 12 carbon atoms or one to three of the radicals $R_1$ to $R_4$ also represent a hydrogen atom.

2. 1,8-Dihydroxy-9-(10H)-anthracenones of general formula I as claimed in claim 1, wherein one or two of the radicals $R_1$ to $R_4$, which may be the same or different, represent chlorine or bromine atoms, branched alkyl groups with 3 to 7 carbon atoms or cycloalkyl groups with 5 to 7 carbon atoms and the rest of the radicals $R_1$ to $R_4$ represent hydrogen atoms.

3. 1,8-Dihydroxy-9-(10H)-anthracenones of general formula I as claimed in claim 2, wherein one or two of the radicals $R_1$ to $R_4$, which may be the same or different, represent chlorine or bromine atoms, isopropyl, isopentyl, hexyl-(2) or cyclohexyl groups and the rest of the radicals $R_1$ to $R_4$ represent hydrogen atoms.

4. 4,5-Dichloro-1,8-dihydroxy-9-(10H)-anthracenone.

5. 2-tert.Butyl-1,8-dihydroxy-9-(10H)-anthracenone.

6. 2,7-Di-tert.butyl-1,8-dihydroxy-9-(10H)-anthracenone.

7. 1,8-Dihydroxy-4-isopropyl-9-(10H)-anthracenone.

8. Pharmaceutical compositions containing one or more compounds of general formula I as claimed in claim 1, besides the usual carriers and excipients.

9. The use of compounds of general formula I as claimed in claim 1, for the preparation of pharmaceutical compositions by a non-chemical method.

10. Pharmaceutical compositions as claimed in claim 8 for the treatment of skin diseases such as psoriasis, dandruff, ichthyosis and hyperkeratotic skin conditions.

11. A process for the preparation of new substituted 1,8-dihydroxy-9-(10H)-anthracenones of general formula I,

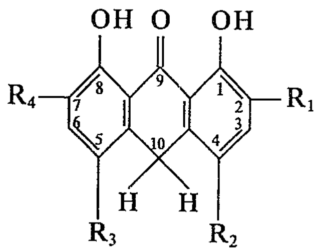

(I)

wherein
$R_1$ to $R_4$, which may be the same or different, represent halogen atoms, branched alkyl groups or cycloalkyl groups each with 3 to 12 carbon atoms or one to three of the radicals $R_1$ to $R_4$ also represent a hydrogen atom, characterised in that

a) in order to prepare compounds of general formula I wherein one of the radicals $R_1$ to $R_4$ represents a halogen atom and the other of these radicals represents a branched alkyl group or a saturated cycloalkyl group or one or two of the radicals $R_1$ to $R_4$ represent a branched alkyl group or a saturated cycloalkyl group each with 3 to 12 carbon atoms and the remaining radicals represent hydrogen atoms, a 1,8-dihydroxy-9-(10H)-anthracenone of general formula II,

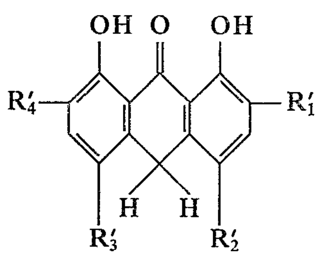

(II)

wherein
$R_1'$ to $R_4'$ represent hydrogen atoms or one of the radicals $R_1'$ to $R_4'$ represents a halogen atom, a branched alkyl group or a cycloalkyl group each with 3 to 12 carbon atoms and the remaining radicals $R_1'$ to $R_4'$ represent hydrogen atoms, is reacted with alkenes, cycloalkenes, alkanols or cycloalkanols each with 3 to 12 carbon atoms, at temperatures between 30 and 150°C in the presence of acidic agents in a solvent, or

b) in order to prepare compounds of general formula I wherein one to four of the radicals $R_1$ to $R_4$ represent halogen atoms and the remaining radicals are as hereinbefore defined, a 1,8-dihydroxy-9-(10H)-anthracenone of general formula II,

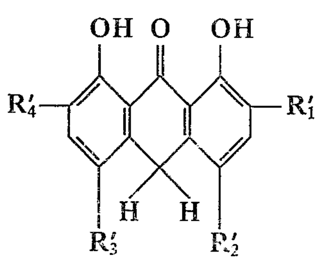

(II)

wherein
$R_1'$ to $R_4'$ represent hydrogen atoms or 1 to 3 of the radicals $R_1'$ to $R_4'$ have the meanings mentioned

hereinbefore for $R_1$ to $R_4$ and the remaining radicals $R_1'$ to $R_4'$ represent hydrogen atoms, is halogenated in a solvent.

12. A process as claimed in claim 11b, characterised in that free halogen, sulphuryl halides or hypohalites are used as halogenating agents.

**Revendications**

1. 1,8-dihydroxy-9-(10H)anthracénones substituées de formule générale I,

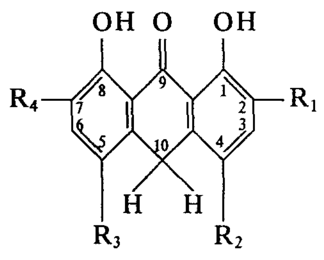

(I)

dans laquelle:
$R_1$ à $R_4$, qui peuvent être identiques ou différents, représentent des atomes d'halogène, des groupes alcoyle ramifiés ou des groupes cycloalcoyle contenant respectivement 3 à 12 atomes de carbone ou bien un à trois des radicaux $R_1$ à $R_4$ représentent aussi un atome d'hydrogène.

2. 1,8-dihydroxy-9-(10H)anthracénones de formule générale I selon la revendication 1, dans laquelle un ou deux des radicaux $R_1$ à $R_4$, qui peuvent être identiques ou différents, représentent des atomes de chlore ou de brome, des groupes alcoyle ramifiés contenant 3 à 7 atomes de carbone ou des groupes cycloalcoyle contenant 5 à 7 atomes de carbone, le restant des radicaux $R_1$ à $R_4$ étant constitué par des atomes d'hydrogène.

3. 1,8-dihydroxy-9-(10H)anthracénones de formule générale I selon la revendication 2, dans laquelle un ou deux des radicaux $R_1$ à $R_4$, qui peuvent être identiques ou différents, représentent des atomes de chlore ou de brome, des groupes isopropyle, isopentyle, hexyle(2) ou cyclohexyle, le restant des radicaux $R_1$ à $R_4$ étant constitué par des atomes d'hydrogène.

4. La 4,5-dichloro-1,8-dihydroxy-9-(10H)anthracénone.

5. La 2-tert.butyl-1,8-dihydroxy-9-(10H)anthracénone.

6. La 2,7-di-tert.butyl-1,8-dihydroxy-9-(10H)anthracénone.

7. La 1,8-dihydroxy-4-isopropyl-9-(10H)anthracénone.

8. Médicament renfermant un ou plusieurs composés de formule générale I selon la revendication 1 ainsi que des excipients et adjuvants habituels.

9. Utilisation des composés de formule générale I selon la revendication 1, pour la préparation de médicaments selon une voie non chimique.

10. Médicament selon la revendication 8, pour le traitement des maladies de la peau telles que le psoriasis, les pellicules, les ichthyoses et les états cutanés hyperkératosiques.

11. Procédé pour la préparation de nouvelles 1,8-dihydroxy-9-(10H)anthracénones substituées de formule générale I,

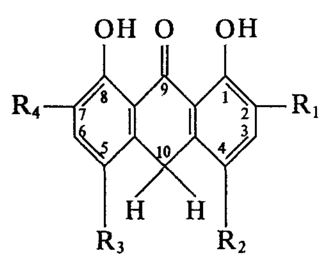

(I)

dans laquelle:
$R_1$ à $R_4$, qui peuvent être identiques ou différents, représentent des atomes d'halogène, des groupes alcoyle ramifiés ou des groupes cycloalcoyle contenant respectivement 3 à 12 atomes de carbone, ou bien un à trois des radicaux $R_1$ à $R_4$ sont également un atome d'hydrogène, caractérisé en ce que:

a) pour préparer les composés de formule générale I, dans laquelle un des radicaux $R_1$ à $R_4$ est un atome d'halogène et un autre de ces radicaux est un groupe alcoyle ramifié ou un groupe cycloalcoyle saturé, ou bien un ou deux des radicaux $R_1$ à $R_4$ est un groupe alcoyle ramifié ou un

groupe cycloalcoyle saturé contenant respectivement 3 à 12 atomes de carbone et les radicaux restants sont des atomes d'hydrogène, on fait réagir une 1,8-dihydroxy-9-(10H)anthracénone de formule générale II,

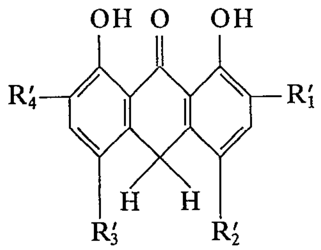

(II)

dans laquelle:
$R_1'$ à $R_4'$ sont des atomes d'hydrogène ou l'un des radicaux $R_1'$ à $R_4'$ est un atome d'halogène, un groupe alcoyle ramifié ou un groupe cycloalcoyle contenant respectivement 3 à 12 atomes de carbone, le restant des radicaux $R_1'$ à $R_4'$ étant constitué d'atomes d'hydrogène, avec des alcènes, des cycloalcènes, des alcanols ou des cycloalcanols contenant respectivement 3 à 12 atomes de carbone, à des températures comprises entre 30 et 150°C et en présence d'agents acides dans un solvant, ou bien

b) pour préparer les composés de formule générale I, dans laquelle un à quatre des radicaux $R_1$ à $R_4$ sont des atomes d'halogène, et les autres radicaux sont tels que définis plus haut, on soumet à une halogénation dans un solvant, une 1,8-dihydroxy-9-(10H)anthracénone de formule générale II,

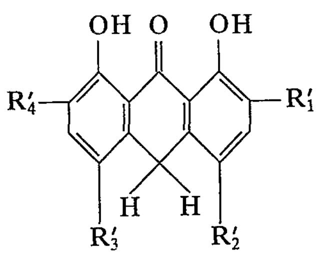

(II)

dans laquelle:
$R_1'$ à $R_4'$ sont des atomes d'hydrogène ou bien 1 à 3 des radicaux $R_1'$ à $R_4'$ possèdent les définitions données plus haut pour $R_1$ à $R_4$, le restant des radicaux $R_1'$ à $R_4'$ étant des atomes d'hydrogène.

12. Procédé selon la revendication 11b), caractérisé en ce qu'on utilise à titre d'agent d'halogénation, un halogène libre, un halogénure de sulfuryle ou un hypohalogénite.

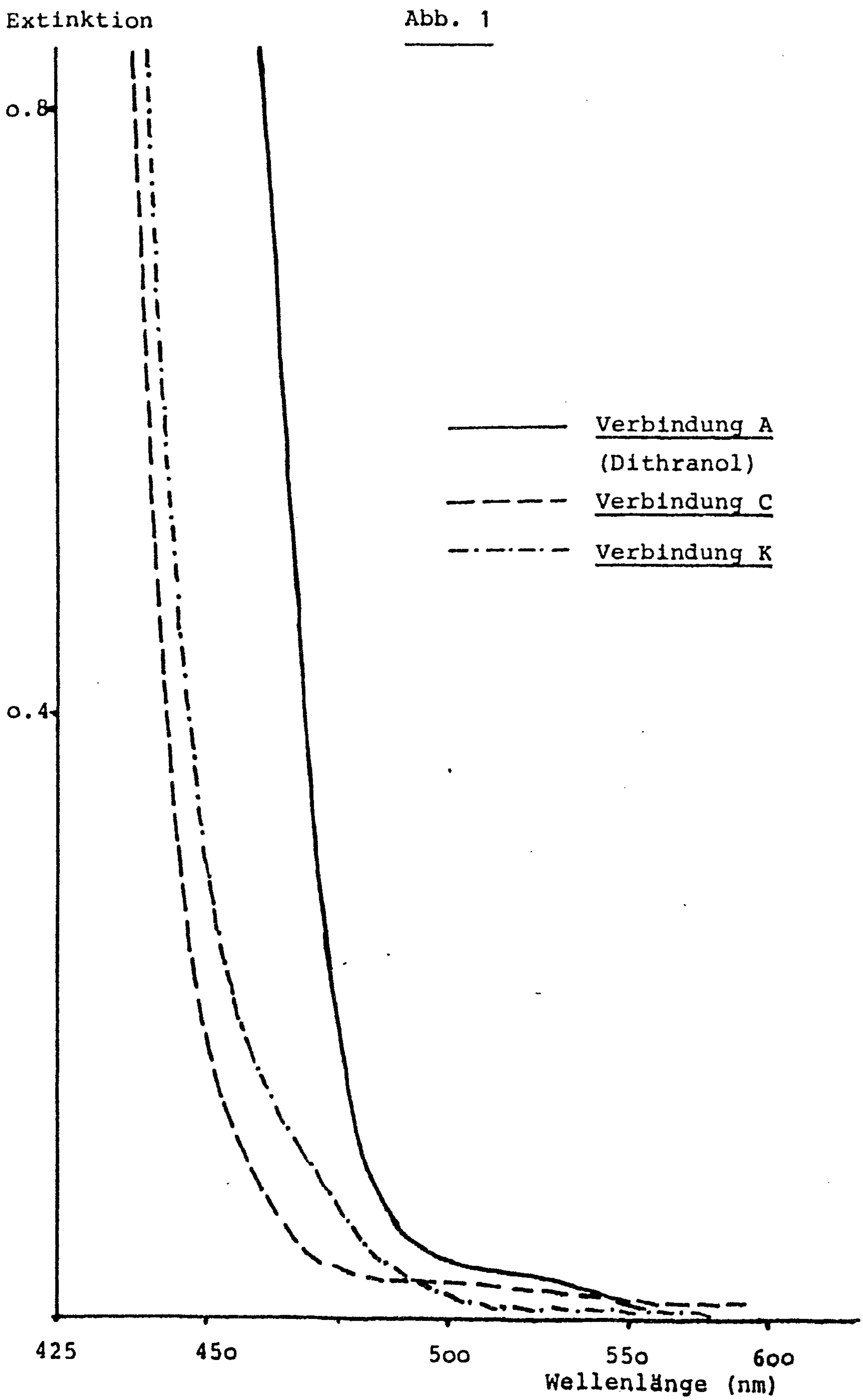
Extinktion
Abb. 1
o.8
o.4
Verbindung A
(Dithranol)
Verbindung C
Verbindung K
425
45o
5oo
55o
6oo
Wellenlänge (nm)